# FASCICULE DE BREVET EUROPEEN

(11) **EP 3 836 873 B1**
(45) Date de publication et mention de la délivrance du brevet: **03.08.2022**
(21) Numéro de dépôt: 19733797.5
(22) Date de dépôt: 28.06.2019
(51) Int. Cl.: A61F 13/08, A61F 13/14

(54) **VETEMENT DE CONTENTION POUR FEMME ENCEINTE, ET SON PROCEDE DE FABRICATION**
STÜTZBEKLEIDUNG FÜR SCHWANGERE FRAUEN UND VERFAHREN ZU IHRER HERSTELLUNG
COMPRESSION CLOTHING FOR PREGNANT WOMEN, AND ITS MANUFACTURING PROCESS

(30) Priorité: 16.08.2018 FR 1857513
(43) Date de publication de la demande: 23.06.2021
(73) Titulaire: Sigvaris AG, 9014 St. Gallen (CH)
(72) Inventeur: MESMIN, Coline, 42610 SAINT ROMAIN LE PUY (FR)
(74) Mandataire: Cabinet Laurent & Charras
(86) Numéro de dépôt international: PCT/EP2019/067315
(87) Numéro de publication internationale: WO 2020/035204

(56) Documents cités:
- WO-A1-97/45080
- WO-A1-2009/114899
- WO-A1-2013/164550
- DE-U1- 29 520 497
- FR-A1- 2 789 271
- JP-B1- 2 963 892
- US-A1- 2016 015 089

## Description

### DOMAINE TECHNIQUE

La présente invention se rapporte au domaine technique des vêtements de contention pour femme enceinte, et plus particulièrement à un vêtement formé par l'assemblage d'un collant de ville ou de compression médicale, et d'une ceinture de maintien.

### ART ANTERIEUR

Dans ce domaine, le document US2016/015089 décrit différents exemples de vêtements comprenant une ceinture et un collant. La ceinture comprend une partie avant et une partie arrière jointes par des coutures latérales. La partie avant comprend des portions de tissu d'épaisseurs et/ou d'élasticité différentes. La partie arrière comprend au moins une portion de tissu réalisée dans un matériau similaire à la partie avant.

L'inconvénient de ce vêtement est la présence des coutures latérales ou ventrales, qui procurent une sensation d'inconfort au contact de la peau, par pincement du ventre de la femme enceinte.

De plus, les coutures latérales limitent le maintien du ventre pesant vers l'avant, ce qui est susceptible de provoquer une douleur au niveau du dos de la femme enceinte. Ces coutures fragilisées par le poids du ventre peuvent d'ailleurs se relâcher avec le temps, déformant la ceinture ou provoquant sa déchirure.

Le nombre de couches de tissus constituant la ceinture ajoutent également une difficulté pour la mise en place de la ceinture sur le tour de taille de la femme enceinte.

Enfin, l'assemblage des différentes portions de la ceinture engendre des coûts de fabrication élevés, et limite la possibilité d'offrir un vêtement sur mesure.

### EXPOSE DE L'INVENTION

Le but de la présente invention est de proposer un vêtement de contention pour femme enceinte amélioré, remédiant aux inconvénients ci-dessus.

A cet effet, il a été mis au point un vêtement de contention pour femme enceinte comprenant un collant et une ceinture assemblés l'un avec l'autre, par exemple par une couture élastique. Le collant est une orthèse médicale de compression ou un bas de ville.

Selon l'invention, la ceinture comprend :
- une première bande qui s'étend de manière ininterrompue sur le tour de taille, entre deux extrémités jointes de manière permanente ou de amovible, et qui est destinée à être mise en contact avec la peau, sur le ventre et le dos de la femme enceinte, par enfilage ou par mise en oeuvre de moyens de réglage disposés aux deux extrémités, et
- une deuxième bande réalisée en matériau moins élastique que la première bande, par exemple en polyuréthane, et disposée en renfort sur cette dernière pour soutenir le poids du ventre pesant vers l'avant et ainsi soulager le dos de la femme enceinte.

Ainsi, l'invention permet de fournir un vêtement de contention pour le maintien du ventre au cours de la grossesse, qui soit pratique, confortable, esthétique et peu cher.

Selon un premier mode de réalisation, les deux extrémités de la première bande sont jointes de manière permanente par une unique couture élastique. Pour un meilleur confort, la couture est de préférence disposée dans le dos de la femme enceinte. Cette couture est par exemple verticale, disposée au contact de la peau du dos le long de la colonne vertébrale. Le dos est aussi utilisé comme support permettant à la ceinture, au niveau de la couture, de soutenir le poids de ventre et de soulager en conséquence le dos de la femme enceinte. Les points de couture ne risquent donc pas de s'user ou de se défaire après une ou plusieurs utilisations de la ceinture.

Selon un deuxième mode de réalisation, les deux extrémités de la première bande sont jointes de manière amovible par les moyens de réglage, par exemple sous forme de pattes de fixation munies de systèmes auto-agrippant, permettant d'ajuster la ceinture autour de la taille de la femme enceinte. Dans cette configuration, la mise en place de la ceinture autour de la taille est simple et rapide. De plus, l'évolutivité de la première bande au cours de la grossesse dépend du réglage de la ceinture, ajustée par la femme enceinte pour son confort.

De préférence, la première bande comprend une portion principale moins élastique qu'une bordure supérieure. De cette manière, la portion principale permet une certaine évolutivité de la première bande au cours de la grossesse (hauteur utérine). La bordure supérieure permet quant à elle de maintenir le vêtement de contention autour du corps de la femme enceinte.

Avantageusement, La portion principale comprend des fibres d'élasthanne et des fibres d'au moins un autre matériau moins élastique que l'élasthanne, par exemple du polyamide ou du polyester. Les différentes fibres ont une masse surfacique variant entre 50 et 500 g/m2. De préférence, la portion principale (33) a une masse surfacique comprise entre 200 et 300 g/m2. La bande supérieure quant à elle comprend par exemple des fibres d'élasthanne.

De préférence, les fibres de la portion principale et de la bordure supérieure sont tricotées ensemble.

Selon un mode de réalisation particulier, la deuxième bande comprend des trous formant une dentelle. Ainsi, différents motifs peuvent être utilisés pour décorer la deuxième bande. De plus, les trous augmentent l'élasticité de la deuxième bande, et donc améliorent l'évolutivité de la ceinture au cours de la grossesse.

Un autre but de l'invention est de proposer un procédé simple et peu coûteux pour la fabrication du vêtement de contention, pouvant s'adapter à la morphologie de la femme enceinte.

A cet effet, il a été mis au point un procédé de fabrication d'un vêtement de contention pour femme enceinte comprenant un collant et une ceinture assemblés l'un avec l'autre conformément à ce qui précède.

Selon l'invention, le procédé comprend les étapes suivantes :
- découpe de la première bande, conçue pour s'étendre de manière ininterrompue sur le tour de taille de la femme enceinte, entre ses deux extrémités prévues pour être jointes;
- découpe de la deuxième bande réalisée en matériau moins élastique que la première bande;
- fixation de la deuxième bande sur la première bande;
- assemblage du collant et de la ceinture.

Ainsi, le procédé simplifié permet de réduire les coûts de production, tout en offrant à la femme enceinte le choix d'un vêtement taillé sur mesure.

De préférence, le procédé met en oeuvre une opération de thermocollage à chaud par pressage de la deuxième bande sur la première bande. Cette opération peut être qualifiée de transfert à chaud, ou de marquage sur textile d'un film mince.

Après l'opération de thermocollage, la ceinture peut être laminée entre deux cylindres chauffés d'une calandre, par exemple avec deux passages de la ceinture entre les cylindres, à une vitesse de 0,6m/min et à une température de 175°C. Ainsi, la ceinture est plus résistante aux lavages.

### BREVE DESCRIPTION DES DESSINS

L'invention sera mieux comprise à la lecture de la description qui va suivre, donnée uniquement à titre d'exemple non limitatif et faite en référence aux dessins annexés sur lesquels :
- la figure 1 est une vue de face d'une femme enceinte portant un vêtement de contention conforme à un premier mode de réalisation de l'invention ;
- la figure 2 est une vue de profil de la femme enceinte avec le vêtement de la figure 1 ;
- la figure 3 est une vue arrière de la femme enceinte avec le vêtement de la figure 1 ;
- la figure 4 est une vue développée de la première bande constituant la ceinture des figures 1 à 3;
- la figure 5 est une vue développée de la ceinture des figures 1 à 3, avec la deuxième bande thermocollée sur la première bande ;
- la figure 6 est une vue de face d'une femme enceinte portant un vêtement de contention conforme à un deuxième mode de réalisation de l'invention ;
- la figure 7 est une vue de profil de la femme enceinte avec le vêtement de la figure 6 ; et
- la figure 8 est une vue arrière de la femme enceinte avec le vêtement de la figure 6 ;

### EXPOSE DETAILLE DE L'INVENTION

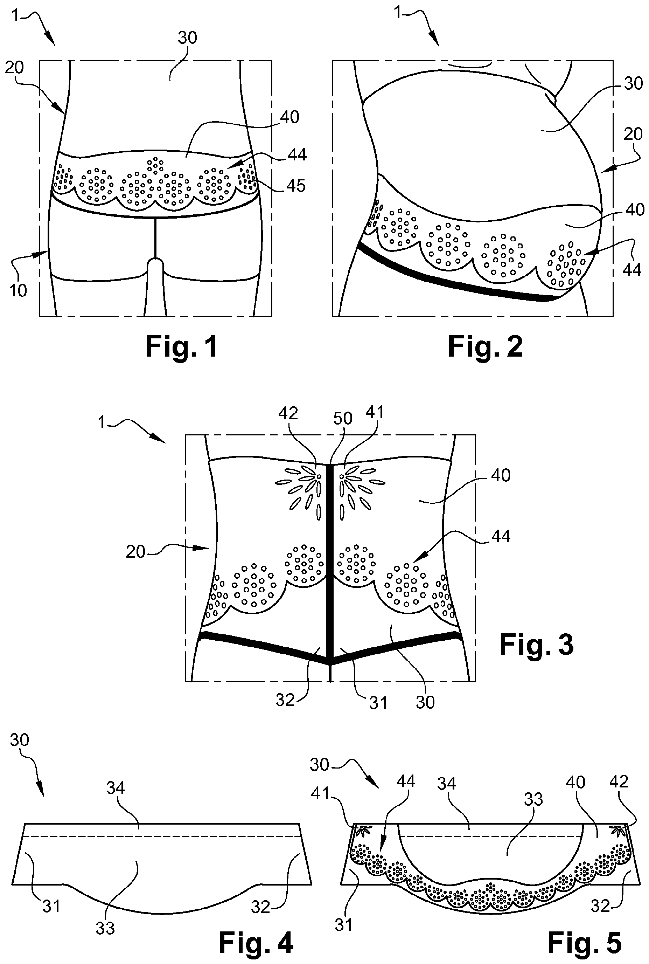

Sur les figures 1 à 5 est représenté un vêtement de contention (1) conforme à l'invention. Le vêtement (1) comprenant un collant (10) et une ceinture (20) assemblés l'un avec l'autre, par exemple par une couture élastique horizontale suivant le tour de taille.

Le collant (10) peut être une orthèse médicale de compression, un bas de ville, un collant de sport (de compression ou non), ou un collant de compression de confort (collant non médical, destiné aux personnes dont les jambes sont en bonne santé, mais qui ont passagèrement des problèmes veineux dans certaines situations, tels que les longs déplacements en avion, grossesse, station assise ou debout prolongée au travail). Le collant (10) peut être avec pieds, sans pieds ou avec pieds ouverts. De préférence, le collant (10) est du type taille basse.

La ceinture (20) comprend deux bandes (30, 40) superposées dans le sens de l'épaisseur. Chaque bande (30, 40) est de préférence monocouche. La bande (40) peut être fixée sur la bande (30) par thermocollage, ou par toute autre technique adaptée à l'application visée.

La bande (30) du dessous est destinée à être mise en contact avec la peau, sur le ventre et le dos de la femme enceinte, par enfilage. La bande (30) est découpée pour s'adapter à la morphologie de la femme enceinte. La bande (30) s'étend de manière ininterrompue sur le tour de taille, entre deux extrémités (31, 32) jointes par une couture élastique (50) verticale disposée dans le dos.

La bande (30) comprend une portion principale (33) et une bordure supérieure (34), de préférence constituées de fibres élastiques tricotées ensemble. Ainsi, la bande (30) forme une unique couche de matière. La bordure supérieure (34) est plus élastique que la portion principale (33) et permet d'assurer le maintien du vêtement (1) autour du corps de la femme enceinte.

De préférence, la bordure supérieure (34) comprend uniquement des fibres d'élasthanne, tandis que la portion principale (33) comprend des fibres d'élasthanne et d'au moins un autre matériau moins élastique que l'élasthanne, par exemple du polyamide ou du polyester. Encore de préférence, les différentes fibres de la portion principale (33) ont une masse surfacique variant entre 50 et 500 g/m2. Toujours de préférence, la portion principale (33) présente une masse surfacique comprise entre 200 et 300 g/m2. Toujours de préférence, la bande (40) a une épaisseur inférieure ou égale à 180 µm.

La bande (40) du dessus est disposée en renfort sur la bande (30), pour soutenir le poids du ventre vers l'avant, et ainsi soulager le dos de la femme enceinte. La bande (40) forme un arc de cercle, qui s'étend en partie basse de la bande (30) sur l'avant de la ceinture (20), afin de soutenir le ventre par le dessous. Pour assurer sa fonction de renfort, la bande (40) est réalisée en matériau moins élastique que la bande (30). La bande (40) est un film ou un transfert formant une unique couche de matière. De préférence, la bande (40) est en polyuréthane. Encore de préférence, la bande (40) a une épaisseur inférieure ou égale à 180 µm.

Comme montré aux figures 3 et 5, la bande (40) s'étend de manière ininterrompue entre deux extrémités (41, 42) voisines des deux extrémités (31, 32) de la bande (30). Cela procure un meilleur support dorsal et ventral.

La bande (40) comprend des trous (45) ménagés à travers son épaisseur, agencés selon des motifs formant une dentelle (44). Les trous (45) permettent d'améliorer l'élasticité et l'esthétique de la ceinture (30).

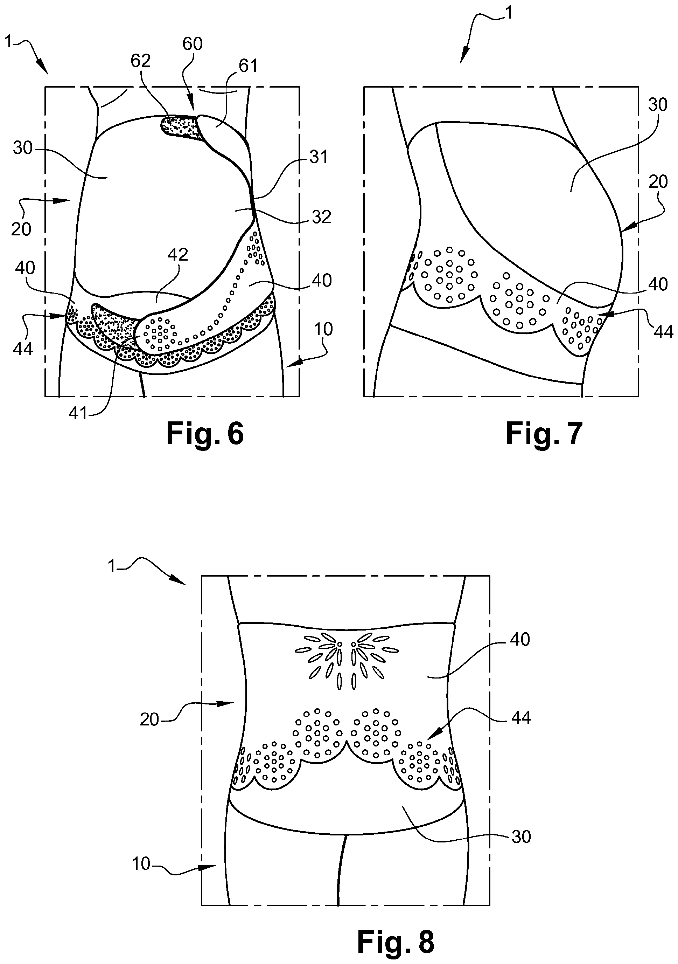

Sur les figures 6 à 8 est représenté un vêtement de contention (1) conforme à un deuxième mode de réalisation de l'invention.

Certains éléments constitutifs de ce vêtement (1) sont comparables à ceux du premier mode de réalisation décrit plus haut et, dans un but de simplification, portent les mêmes références numériques.

Dans ce deuxième mode de réalisation, les deux extrémités (31, 32) de la bande (30) sont jointes de manière amovible par des moyens de réglage (60), permettant d'ajuster la ceinture (20) autour de la taille de la femme enceinte.

Plus précisément, les moyens de réglage (60) sont par exemple des systèmes auto-grippant type scratch. Ainsi, des pattes de fixation (61) formées à l'extrémité (31) de la bande (30) sont équipées de crochets prévus pour venir se fixer sur des parties « velours » (62) de la ceinture (20), disposées du côté de l'extrémité (32). Les pattes (61) de la bande (30) forment par exemple un « C » pour permettre la fixation autour du ventre de la femme enceinte, en dessus et en dessous. La patte (61) qui vient se fixer sur le bas ventre est également renforcée sur sa surface extérieure par la bande (40).

Ainsi, en ajustant la ceinture (20) autour de la taille, le maintien du bas ventre au cours de la grossesse est aussi garanti.

En alternative, les moyens de réglage (60) peuvent comporter des agrafes de fixation, ou tout autre système adapté à l'application visée.

Par ailleurs, le vêtement (1) peut être conformé différemment des figures 1 à 8 sans sortir du cadre de l'invention.

En outre, les caractéristiques techniques des différents modes de réalisation et variantes mentionnés ci-dessus peuvent être, en totalité ou pour certaines d'entre elles, combinées entre elles.

Ainsi, le vêtement (1) peut être adapté en termes de coût, de fonctionnalités et de performance.

Il ressort de ce qui précède que l'invention fournit bien un vêtement de contention (1) pour le maintien du ventre au cours de la grossesse qui soit pratique, confortable, esthétique et peu cher.

## Revendications

1. Vêtement de contention (1) pour femme enceinte, comprenant un collant (10) et une ceinture (20) assemblés l'un avec l'autre, le collant (10) étant une orthèse médicale de compression, un bas de ville, un collant de sport, ou un collant de compression de confort, ***caractérisé en ce que*** la ceinture (20) comprend :
- une première bande (30) qui s'étend de manière ininterrompue sur le tour de taille entre deux extrémités (31, 32) jointes, et qui est destinée à être mise en contact avec la peau, sur le ventre et le dos de la femme enceinte, par enfilage ou par mise en oeuvre de moyens de réglage (60) disposés aux deux extrémités (31, 32), et
- une deuxième bande (40) réalisée en matériau moins élastique que la première bande (30) et disposée en renfort sur cette dernière pour soutenir le ventre pesant vers l'avant et ainsi soulager le dos de la femme enceinte.

2. Vêtement de contention (1) selon la revendication 1, ***caractérisé en ce que*** la première bande (30) comprend une portion principale (33) et une bordure supérieure (34), qui est plus élastique que la portion principale (33) et qui permet d'assurer le maintien du vêtement (1) autour du corps de la femme enceinte.

3. Vêtement de contention (1) selon la revendication 2, ***caractérisé en ce que*** la portion principale (33) et la bordure supérieure (34) sont constituées de fibres élastiques tricotées ensemble.

4. Vêtement de contention (1) selon l'une quelconque des revendications 2 ou 3, ***caractérisé en ce que*** la portion principale (33) comprend des fibres d'élasthanne et des fibres d'au moins un autre matériau moins élastique que l'élasthanne, par exemple du polyamide ou du polyester, les différentes fibres ayant une masse surfacique variant entre 50 et 500 g/m2.

5. Vêtement de contention (1) selon l'une quelconque des revendications 2 à 4, ***caractérisé en ce que*** la bordure supérieure (34) comprend uniquement des fibres d'élasthanne.

6. Vêtement de contention (1) selon l'une des revendications précédentes, ***caractérisé en ce que*** la deuxième bande (40) est en polyuréthane.

7. Vêtement de contention (1) selon l'une quelconque des revendications précédentes, ***caractérisé en ce que*** la deuxième bande (40) comprend des trous (45) formant une dentelle (44).

8. Vêtement de contention (1) selon l'une quelconque des revendications 1 à 8, ***caractérisé en ce que*** les deux extrémités (31, 32) de la première bande (30) sont jointes de manière permanente par une unique couture élastique (50).

9. Vêtement de contention (1) selon l'une quelconque des revendications 1 à 8, ***caractérisé en ce que*** les deux extrémités (31, 32) de la première bande (30) sont jointes de manière amovible par les moyens de réglage (60), permettant d'ajuster la ceinture (20) autour de la taille de la femme enceinte.

10. Procédé de fabrication d'un vêtement de contention (1) pour femme enceinte, comprenant un collant (10) et une ceinture (20) assemblés l'un avec l'autre, le vêtement (1) étant conforme à l'une quelconque des revendications précédentes, ***caractérisé en ce que*** le procédé comprend les étapes suivantes :
- découpe de la première bande (30), conçue pour s'étendre de manière ininterrompue sur le tour de taille de la femme enceinte, entre ses deux extrémités (31, 32) prévues pour être jointes ;
- découpe de la deuxième bande (40) réalisée en matériau moins élastique que la première bande (30) ;
- fixation de la deuxième bande (40) sur la première bande (30) ;
- assemblage du collant (10) et de la ceinture (20).

## Patentansprüche

1. Stützende Kleidung (1) für Schwangere, bestehend aus einer Strumpfhose (10) und einem Bund (20), die miteinander verbundenen sind, bei der Strumpfhose (10) handelt es sich um eine medizinische Kompressionsorthese, handelsübliche Strümpfe, eine Sportstrumpfhose oder eine Komfort- Kompressions- Strumpfhose, ***dadurch gekennzeichnet, dass*** der Bund (20) umfasst:
- ein erstes Band (30), das zwischen zwei verbundenen Endstücken (31, 32) ununterbrochen um die Taille verläuft und dabei auf dem Bauch und dem Rücken der Schwangeren in Kontakt zur Haut kommt, durch Einfädeln oder durch Verwendung von Einstellmitteln (60), die in den beiden Endstücken (31,32) vorgesehen sind, und
- ein zweites Band (40), ausgeführt aus weniger elastischem Material als das erste Band (30) und angeordnet zu dessen Verstärkung um den nach vorne lastenden Bauch zu stützen und so den Rücken der Schwangeren zu entlasten.

2. Stützende Kleidung (1) nach Anspruch 1, ***dadurch gekennzeichnet, dass*** das erste Band (30) einen Hauptabschnitt (33) und eine obere Randbordüre (34) enthält, die elastischer ist als der Hauptabschnitt (33) und die den Halt des Kleidungsstücks (1) am Körper der Schwangeren gewährleistet.

3. Stützende Kleidung (1) nach Anspruch 2, ***dadurch gekennzeichnet, dass*** der Hauptabschnitt (33) und die obere Bordüre (34) aus miteinander verstrickten elastischen Fasern besteht.

4. Stützende Kleidung (1) nach einem beliebigen der Ansprüche 2 oder 3, ***dadurch gekennzeichnet, dass*** der Hauptabschnitt (33) aus Elasthanfasern und Fasern aus mindestens einem anderen, weniger elastischem Material als Elasthan besteht, zum Beispiel Polyamid oder Polyester, die verschiedenen Fasern haben dabei ein Flächengewicht zwischen 50 und 500 g/m2.

5. Stützende Kleidung (1) nach einem beliebigen der Ansprüche 2 bis 4, ***dadurch gekennzeichnet, dass*** die obere Bordüre (34) ausschließlich Elasthanfasern enthält.

6. Stützende Kleidung (1) nach einem der vorangehenden Ansprüche, ***dadurch gekennzeichnet, dass*** das zweite Band (40) aus Polyurethan besteht.

7. Stützende Kleidung (1) nach irgendeinem der vorangehenden Ansprüche, ***dadurch gekennzeichnet, dass*** das zweite Band (40) Löcher (45) enthält, die eine Spitze (44) bilden.

8. Stützende Kleidung (1) nach irgendeinem der vorangehenden Ansprüche 1 bis ***8, dadurch gekennzeichnet, dass*** die beiden Endstücke (31, 32) des ersten Bandes (30) permanent durch eine einzige elastische Naht (50) verbunden sind.

9. Stützende Kleidung (1) nach irgendeinem der vorangehenden Ansprüche 1 bis ***8, dadurch gekennzeichnet, dass*** die beiden Endstücke (31, 32) des ersten Bandes (30) lösbar durch Einstellmittel (60) verbunden sind, mit denen der Bund (20) um die Taille der Schwangeren eingestellt werden kann.

10. Herstellungsverfahren für ein stützendes Kleidungsstück (1) für Schwangere, mit einer Strumpfhose (10) und einem Bund (20) die miteinander verbunden sind, das Kleidungsstück (1) entspricht dabei einem beliebigen der vorhergehenden Ansprüche, ***dadurch gekennzeichnet, dass*** das Verfahren die folgenden Schritte enthält:
- Schneiden des ersten Bandes (30), entwickelt um ununterbrochen um den Taillenumfang der Schwangeren zu führen, zwischen zwei Endstücken (31, 32), die dazu vorgesehen sind, miteinander verbunden zu werden;
- Schneiden des zweiten Bandes (40), hergestellt aus weniger elastischem Material als das erste Band (30);
- Befestigung des zweiten Bandes (40) am ersten Band (30);
- Verbindung der Strumpfhose (10) und des Bundes (20).

## Claims

1. A support garment (1) for a pregnant woman, comprising tights (10) and a belt (20) assembled together, the tights (10) being a medical compression orthosis, everyday tights, sport tights or comfort compression tights, ***characterized in that*** the belt (20) comprises:
- a first band (30) which extends around the waist in an uninterrupted manner between two ends (31, 32) and which is intended to come into contact with the skin, on the abdomen and the back of the pregnant woman, by putting it on or by employing adjustment means (60) disposed at the two ends (31, 32), and
- a second band (40) made of a material that is less elastic than the first band (30) and disposed on the first band as a reinforcement in order to support the weight of the forwardly swelling abdomen and thus relieve the back of the pregnant woman.

2. The support garment (1) as claimed in claim 1, ***characterized in that*** the first band (30) comprises a principal portion (33) and an upper edge (34) which is more elastic than the principal portion (33) and which can ensure that the garment (1) is held around the body of the pregnant woman.

3. The support garment (1) as claimed in claim 2, ***characterized in that the*** principal portion (33) and the upper edge (34) are constituted by elastic fibres knitted together.

4. The support garment (1) as claimed in either claim 2 or claim 3, ***characterized in that the*** principal portion (33) comprises fibres of elastane and fibres of at least one other material which is less elastic than the elastane, for example polyamide or polyester, the various fibres having a surface mass in the range 50 to 500 g/m².

5. The support garment (1) as claimed in any one of claims 2 to 4, ***characterized in that the*** upper edge (34) solely comprises elastane fibres.

6. The support garment (1) as claimed in any one of the preceding claims, ***characterized in that*** the second band (40) is made of polyurethane.

7. The support garment (1) as claimed in any one of the preceding claims, ***characterized in that*** the second band (40) comprises holes (45) forming a lacework (44).

8. The support garment (1) as claimed in any one of claims 1 to 8, ***characterized in that*** the two ends (31, 32) of the first band (30) are permanently joined by means of a single elastic seam (50).

9. The support garment (1) as claimed in any one of claims 1 to 8, ***characterized in that*** the two ends (31, 32) of the first band (30) are joined in a detachable manner via adjustment means (60) allowing the belt (20) to be adjusted around the waist of the pregnant woman.

10. A method for the manufacture of a support garment (1) for a pregnant woman, comprising tights (10) and a belt (20) assembled together, the garment (1) being as claimed in any one of the preceding claims, ***characterized in that*** the method comprises the following steps:
- cutting out the first band (30), designed to extend in an uninterrupted manner around the waist of the pregnant woman between its two ends (31, 32) which are meant to be joined;
- cutting out the second band (40) made of a material which is less elastic than the first band (30);
- fastening the second band (40) to the first band (30);
- assembling the tights (10) and the belt (20).
